# EUROPEAN PATENT APPLICATION

(11) **EP 4 455 269 A1**
(43) Date of publication of application: **30.10.2024**
(21) Application number: 22912032.4
(22) Date of filing: 23.12.2022
(51) Int. Cl.: C12N 5/071, A61K 35/36, A61P 17/02, A61K 8/98, A61Q 19/00

(54) **METHOD FOR CULTURING SKIN-DERIVED STEM CELLS, AND USE THEREOF**

(30) Priority: 24.12.2021 KR 20210187681
(71) Applicant: Ewha University-Industry Collaboration Foundation, Seoul 03760 (KR); Hierabio Inc., Seoul 03760 (KR)
(72) Inventor: LEE, Seung Jin, Seoul 07333 (KR); SHIN, Ji-young, Gyeryong-si, Chungcheongnam-do 32800 (KR); JEON, Jeong Hwa, Seoul 03938 (KR); KIM, Heejung, Seongnam-si, Gyeonggi-do 13503 (KR)
(74) Representative: Studio Torta S.p.A.
(86) International application number: PCT/KR2022/021212
(87) International publication number: WO 2023/121399

(57) **Abstract**

The present invention relates to a method for culturing skin-derived stem cells and uses of the same, particularly to a method for culturing skin-derived stem cells; skin-derived stem cells prepared by the method; and a pharmaceutical composition for skin regeneration or wound treatment; a quasi-drug composition; and a cosmetic composition, which contain the prepared skin-derived stem cells, a culture of the skin-derived stem cells, or cells differentiated from the skin-derived stem cells as an active ingredient.

According to the culture method of the present invention, stem cells can be effectively isolated and obtained from skin tissue, and the obtained skin-derived stem cells have excellent pluripotency and self-replication ability as well as excellent skin regeneration and wound treatment effects and can be usefully utilized for skin regeneration and wound treatment or improvement.

## Description

### [Technical Field]

The present invention relates to a method for culturing skin-derived stem cells and uses thereof, particularly to a method for culturing skin-derived stem cells; skin-derived stem cells prepared by the method; and a pharmaceutical composition for skin regeneration or wound treatment; a quasi-drug composition; and a cosmetic composition, which contain the prepared skin-derived stem cells, a culture of the skin-derived stem cells, or cells differentiated from the skin-derived stem cells as an active ingredient.

### [Background Art]

Stem cells are cells in a stage before differentiating into the respective cells that make up tissue, and are cells that can proliferate indefinitely in an undifferentiated state and have the potential to differentiate into cells of various tissues by specific differentiation stimulation.

Stem cells are largely divided into embryonic stem cells (ES cells) and adult stem cells (tissue specific stem cells) depending on the differentiation potential. Embryonic stem cells are stem cells isolated from the inner cell mass (ICM), which will develop into a fetus, of a blastocyst embryo, the initial stage in which the fertilized egg is formed but is not yet implanted in the endometrium, and are cells that have the potential to differentiate into cells of all tissues.

Meanwhile, tissue specific stem cells are stem cells specific to each organ that appear in the stage in which the embryonic development process progresses and each organ of the embryo is formed, and their differentiation potency is generally limited (multipotent) only to the cells that make up the tissue. Representative tissue specific stem cells include hematopoietic stem cells, which exist in bone marrow, and mesenchymal stem cells, which differentiate into connective tissue cells other than blood cells. Hematopoietic stem cells differentiate into various blood cells such as red blood cells and white blood cells, and mesenchymal stem cells differentiate into osteoblasts, chondroblasts, adipocytes, and myoblasts.

Recently, since the success of the isolation of embryonic stem cells from humans, interest in their clinical application has increased. The application of stem cells that is receiving the most attention is the use of stem cells as a cell source for cell replacement therapy.

### [Disclosure]

### [Technical Problem]

The existing skin stem cell isolation method is an enzymatic isolation method using collagenase and the like (Korean Patent Publication No. 1 0-2014-0075469 A), which has the disadvantage of affecting cell performance since the damage to proteins on the cell surface is inevitable, and it is thus required to study skin stem cell isolating and culturing methods.

Against this background, the present inventors have conducted various studies to develop new stem cells, as a result, found out that skin-derived stem cells prepared by the culture method of the present invention have excellent in vitro proliferation ability, cell performance, and differentiation potency, and can be used for skin regeneration and wound treatment, and thus completed the present invention.

### [Technical Solution]

An object of the present invention is to provide a method for culturing skin-derived stem cells using skin tissue and a hydrogel.

Another object of the present invention is to provide skin-derived stem cells prepared by the method.

Still another object of the present invention is to provide a pharmaceutical composition for skin regeneration or wound treatment, containing the skin-derived stem cells, a culture of the skin-derived stem cells, or cells differentiated from the skin-derived stem cells as an active ingredient.

Still another object of the present invention is to provide a quasi-drug composition for skin regeneration or wound improvement, containing the skin-derived stem cells, a culture of the skin-derived stem cells, or cells differentiated from the skin-derived stem cells as an active ingredient.

Still another object of the present invention is to provide a cosmetic composition for skin regeneration or wound improvement, containing the skin-derived stem cells, a culture of the skin-derived stem cells, or cells differentiated from the skin-derived stem cells as an active ingredient.

Still another object of the present invention is to provide a composition for skin-derived stem cell culture, containing skin tissue and a hydrogel.

Still another object of the present invention is to provide a kit for skin-derived stem cell culture, containing the composition for stem cell culture.

### [Advantageous Effects]

According to the culture method of the present invention, stem cells can be effectively isolated and obtained from skin tissue, and the obtained skin-derived stem cells have excellent pluripotency and self-replication ability as well as excellent skin regeneration and wound treatment effects and can be usefully utilized for skin regeneration and wound treatment or improvement.

### [Brief Description of Drawings]

FIG. 1 illustrates the results of tissue culture of HB-skin-derived stem cells of the present invention (FIG. 1A) and the results of cell isolation and culture (FIG. 1B);
FIG. 2 illustrates the results of examining the immunophenotype of HB-skin-derived stem cells of the present invention;
FIG. 3 illustrates the results of examining the proliferation ability of HB-skin-derived stem cells of the present invention when subculture is performed;
FIG. 4 illustrates the results of examining the colony formation ability of HB-skin-derived stem cells (HB-SSC) of the present invention;
FIG. 5A illustrates the results of examining the differentiation potency of HB-skin-derived stem cells of the present invention into adipocytes;
FIG. 5B illustrates the results of examining the differentiation potency of HB-skin-derived stem cells of the present invention into osteocytes;
FIG. 6A illustrates the results of DB-based skin regeneration function-related gene String Network analysis and the results of identifying specific skin regeneration functional genes of HB-skin-derived stem cells (HB-SSC);
FIG. 6B illustrates the comparative analysis results of the expression levels of skin regeneration functional genes specifically expressed in HB-skin-derived stem cells (HB-SSC) of the present invention compared to those in adipose-derived stem cells (ASC);
FIG. 6C illustrates the comparative analysis results of the expression levels of skin regeneration functional genes specifically expressed in HB-skin-derived stem cells (HB-SSC) of the present invention compared to those in adipose-derived stem cells (ASC);
FIG. 7A illustrates the results of examining the wound healing effect of HB-skin-derived stem cells (HB-SSC) of the present invention in a mouse model;
FIG. 7B illustrates the results of examining the wound healing effect of HB-skin-derived stem cells (HB-SSC) of the present invention in a mouse model; and
FIG. 8 illustrates the results of examining the skin and skin structure regeneration ability of HB-skin-derived stem cells (HB-SSC) of the present invention in a mouse wound model.

### [Detailed Description of Preferred Embodiments]

Hereinafter, the present invention will be described in detail. Meanwhile, each description and each embodiment disclosed in the present invention may also be applied to other descriptions and other embodiments, respectively. That is, all combinations of various elements disclosed in the present invention fall within the scope of the present invention. Further, the scope of the present invention is not limited by the following specific description.

An aspect of the present invention to achieve the objects provides a method for culturing skin-derived stem cells using skin tissue and a hydrogel, and skin-derived stem cells cultured by the method.

In the present invention, the term "skin-derived stem cells" may include all stem cells derived from skin tissue, for example, may include adipose stem cells, epidermal stem cells, and dermal stem cells, but is not limited thereto.

Specifically, the method for culturing skin tissue-derived stem cells includes (a) encapsulating skin tissue in a hydrogel and culturing the skin tissue to obtain a culture; and (b) decomposing the hydrogel in the obtained culture to recover stem cells that have migrated from the skin tissue into the hydrogel and proliferated in the hydrogel.

In the present invention, the hydrogel refers to a three-dimensional network structure formed by crosslinking hydrophilic polymers through covalent or noncovalent bonds. As skin tissue is three-dimensionally encapsulated in a hydrogel, the hydrogel may provide physical support to the skin tissue, and at the same time, provide an extracellular matrix function that allows skin-derived stem cells present in the skin tissues to migrate into and proliferate in the hydrogel.

The hydrogel of the present invention is preferably a phase transitional hydrogel that exists in a solution state and can be converted into sol and gel for hydrogel-supported three-dimensional culture, and may specifically be one or more selected from the group consisting of collagen, gelatin, chondroitin, hyaluronic acid, alginic acid, MatrigelTM, chitosan, peptide, fibrin, PGA (polyglycolic acid), PLA (polylactic acid), PEG (polyethylene glycol), and polyacrylamide, but is not limited thereto. Step (a) may involve encapsulating hair follicle tissue in a multilayered hydrogel and culturing the hair follicle tissue. Step (a) may specifically involve encapsulating hair follicle tissue in a double-layered hydrogel and culturing the hair follicle tissue, but is not limited thereto.

In step (a), the culture of a hydrogel in which skin tissue is encapsulated may be performed after the hydrogel in which skin tissue is encapsulated is immersed in a conventional medium known to be suitable for stem cell culture in the art.

In step (a), the culture of skin tissue may involve encapsulating a skin fragment in a hydrogel and culturing the skin fragment for 3 to 20 days, 3 to 18 days, or 3 to 16 days. The culture may specifically be performed for 3 to 14 days, but is not limited thereto.

In an exemplary embodiment of the present invention, the skin of a healthy adult was collected, skin fragments were formed, and the skin fragments were encapsulated in a hydrogel and cultured for 9 days.

As a pretreatment step of skin tissue before step (a), a step of treating skin tissue sample at a temperature of 50°C to 70°C for 30 to 90 seconds; a step of immersing the skin tissue sample in an alcohol or a surfactant for 1 to 10 minutes; and a step of performing washing may be included.

At this time, the temperature for the skin tissue treating step may be 50°C to 70°C, 52°C to 68°C, 54°C to 66°C, 56°C to 64°C, 58°C to 62°C, or 60°C, but is not limited thereto.

The time for the skin tissue treating step may be 30 seconds to 90 seconds, 40 seconds to 80 seconds, 50 seconds to 70 seconds, 55 seconds to 65 seconds, 58 to 62 seconds, 59 seconds, 60 seconds, or 61 seconds, but is not limited thereto.

The alcohol used in the step of immersing the skin tissue sample in an alcohol or a surfactant during the pretreatment step may include an alcohol having 1 to 4 carbon atoms, may specifically be ethanol, methanol, propanol, isopropyl alcohol, and the like, but is not limited thereto.

The surfactant may include an amphoteric surfactant or a nonionic surfactant.

The amphoteric surfactant may include phosphatidylserine, phosphatidylethanolamine, phosphatidylcholine, and the like, but is not limited thereto.

The nonionic surfactant may include fatty acid esters of glycerol, fatty acid esters of sorbitol, Spans or Tweens, and the like, may specifically include glycerol monostearate, glycerol monolaurate, sorbitan monolaurate, sorbitan monostearate, sorbitan tristearate, Tween 20, Tween 40, Tween 60, Tween 80, and the like, but is not limited thereto.

The step of immersing the skin tissue sample in an alcohol or a surfactant is for removing the fat layer of skin tissue, and includes any method of bringing the tissue into contact with an alcohol or a surfactant by any means used in the art, such as immersion, spraying, or coating, and is not particularly limited.

The time for the step of immersing the skin tissue sample in an alcohol or a surfactant may be 1 minute to 10 minutes, 2 minutes to 9 minutes, 3 minutes to 8 minutes, 4 minutes to 7 minutes, 4 minutes 30 seconds to 6 minutes 30 seconds, 5 minutes to 5 minutes 30 seconds, or 5 minutes, but is not limited thereto.

The washing step in the pretreatment step is only required to be used as a cleaning agent in the present technical field and is not particularly limited.

As a specific example, the cleaning agent may be PBS, and the number of washings may be 1 to 10 times, 2 to 8 times, 3 times, 4 times, 5 times, 6 times, or 7 times, but the washing step is not limited thereto.

The skin tissue sample may be a sample prepared using the skin tissue extracted from an animal or donated from a human, but is not limited thereto.

In Example of the present invention, skin tissue was used as a sample and subjected to a pretreatment step of treating the skin tissue at about 60°C for about 1 minute to isolate the stratum corneum, immersing the skin tissue in isopropyl alcohol for 5 minutes to remove the fat layer of the skin, and then performing washing.

In step (b), only the hydrogel may be selectively decomposed in order to recover skin-derived stem cells that have migrated/proliferated in the hydrogel after the hydrogel-supported three-dimensional culture step of step (a).

The hydrogel may be decomposed through one or more enzymes selected from the group consisting of collagenase, gelatinase, urokinase, streptokinase, TPA (tissue plasminogen activator), plasmin, and hyaluronidase.

The skin stem cell isolation/culture technology of the present invention is a hydrogel-based niche preservation self-replication induced isolation technology, which isolates stem cells, and corresponds to a new technology that allows skin tissue to survive/culture in vitro and to isolate high-purity cells directly from the tissue without enzyme treatment.

Unlike existing methods, this isolation method differs from existing methods and has the advantage of being superior in terms of in vitro proliferation ability and cell performance.

Cells isolated by existing methods can be amplified in vitro only up to 2 to 3 passages (maximum 5 passages), but cells isolated by the method of the present invention have excellent performance that cellular senescence does not occur when the cells are subcultured 8 passages or more and can be subcultured for a long period of time, and mass production of the cells is possible.

It has been found that the skin-derived stem cells prepared by the method maintain the cell shape and the cell size until the ninth subculture after tissue culture and cell isolation.

Meanwhile, skin-derived stem cells prepared by the method for preparing skin-derived stem cells provided by the present invention exhibit immunological characteristics in which CD29, CD44, CD73, CD90, or CD105 is expressed on the cell surface but CD14, CD34, or CD45 is not expressed, and exhibit pluripotent characteristics such that the skin-derived stem cells can differentiate into adipocytes, osteocytes, and the like, but are not limited thereto.

The skin-derived stem cells may be used interchangeably with skin-derived stem cells prepared by the method for preparing skin-derived stem cells provided by the present invention, skin-derived stem cells of the present invention, skin-derived stem cells, HB-skin-derived stem cells, HB-skin-derived stem cells of the present invention, and HB-SSC.

In Example of the present invention, it was possible to verify the proliferation ability, differentiation potency, and colony formation ability of skin-derived stem cells prepared by the method for preparing skin-derived stem cells of the present invention.

In Example of the present invention, it was possible to verify the skin wound treatment effect of skin-derived stem cells prepared by the method for preparing skin-derived stem cells of the present invention.

In Example of the present invention, it was further possible to verify that skin-derived stem cells prepared by the method for preparing skin-derived stem cells of the present invention had a long survival period in vitro and excellent viability through the evaluation of cell growth ability/cellular senescence (in vitro proliferative power) and purity of primary cells when subculture was performed.

Skin-derived stem cells prepared by the method for preparing skin-derived stem cells provided by the present invention can express specific skin regeneration functional genes.

At this time, the skin regeneration functional genes may specifically be one or more genes selected from OSR2 (odd-skipped-related 2), DMKN (dermokine), SBSN (suprabasin), or GAS1 (growth arrest specific 1), but are not limited thereto, and may include any gene known to be a skin regeneration functional gene.

In Example of the present invention, it was found that the expression levels of the four skin regeneration functional genes in skin-derived stem cells prepared by the method for preparing skin-derived stem cells were as remarkable as 10-fold or more compared to those in adipose-derived stem cells, and it was possible to verify that skin-derived stem cells prepared by the method of the present disclosure exhibited specific skin regeneration functional genes.

Another aspect of the present invention provides a pharmaceutical composition for skin regeneration or wound treatment, containing the skin-derived stem cells, a culture of the skin-derived stem cells, or cells differentiated from the skin-derived stem cells as an active ingredient.

In the present invention, the term "skin regeneration" may refer to the recovery process of skin tissue against damage to the skin caused by external and internal causes, and the damage caused by external causes may be damage by ultraviolet rays, damage by pollutants, wounds, trauma, and the like, and internal causes may include genetic and psychological causes, but the skin regeneration is not limited thereto.

The skin regeneration is a concept that may include hair follicle regeneration or proliferation of hair follicle cells, and the proliferation of hair follicle cells may include promoting the conversion of hair follicle cells from the telogen phase to the anagen phase, but the skin regeneration is not limited thereto.

In the present invention, the term "culture" or "culture solution" may refer to a culture obtained during or after culture of skin-derived stem cells in a medium, or its supernatant, its concentrate, or its lyophilized product.

In the present invention, the term "pharmaceutical composition" refers to a product prepared for the purpose of preventing or treating a disease, and may be administered in various oral and parenteral dosage forms in actual clinical administration, and may be prepared using commonly used diluents or excipients such as fillers, extenders, binders, wetting agents, disintegrants, and surfactants in the case of being formulated.

Depending on the formulation, pharmaceutically acceptable additives may be further contained, and at this time, as the pharmaceutically acceptable additives, starch, gelatinized starch, microcrystalline cellulose, lactose, povidone, colloidal silicon dioxide, calcium hydrogen phosphate, lactose, mannitol, taffy, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, and talc may be used. The pharmaceutically acceptable additives according to the present invention may be contained by 0.1 to 90 parts by weight based on the composition.

In addition to the skin-derived mesenchymal stem cells, the pharmaceutical composition may further contain one or more conventional pharmaceutically acceptable inert carriers, for example, preservatives, analgesics, solubilizers, stabilizers or the like in the case of injections and bases, excipients, lubricants, preservatives or the like in the case of preparations for topical administration.

The pharmaceutical composition of the present invention may be administered as an individual therapeutic agent or in combination with other therapeutic agents, and may be administered sequentially or simultaneously with conventional therapeutic agents. The pharmaceutical composition of the present invention may be administered a single time or multiple times. It is important to administer the pharmaceutical composition of the present invention in a minimum amount that can afford the maximum effect as well as does not cause side effects, taking all the factors into consideration, and this minimum amount can be easily determined by a person skilled in the art.

In addition, the pharmaceutical composition provided by the present invention may further contain various ingredients that assist skin regeneration or wound treatment, maintain the activity of the skin-derived stem cells, or promote differentiation of the skin-derived stem cells other than the skin-derived stem cells, as an example, may further contain anti-inflammatory agents, stem cell mobilization factors, growth inducing factors, and the like.

In the present invention, the term "improvement" may refer to any action that delays the progression of skin damage or alleviates symptoms by administering the composition according to the present invention to an individual.

In the present invention, the term "prevention" may refer to any action that suppresses or delays the occurrence of skin damage by administering the composition according to the present invention to an individual.

In the present invention, the term "treatment" may refer to any action that improves or benefits skin damage symptoms by administering the composition of the present invention to an individual suspected of having skin damage.

Still another object of the present invention is to provide a method for skin regeneration or wound improvement or treatment, which includes administering to an individual a composition containing the skin-derived stem cells, a culture of the skin-derived stem cells, or cells differentiated from the skin-derived stem cells as an active ingredient.

The skin-derived stem cells, culture of the skin-derived stem cells, or cells differentiated from the skin-derived stem cells, skin regeneration, wound improvement, and treatment are as described above.

In the present invention, the term "administration" means introducing the composition into an individual in an appropriate manner. Specifically, a composition containing the skin-derived stem cells of the present invention, a culture of the skin-derived stem cells, or cells differentiated from the skin-derived stem cells as an active ingredient, or the pharmaceutical composition containing the same may be applied to a local site, for example, to a damaged or wounded skin site or administered as an injection, but the administration is not limited thereto.

The composition may be used in combination with other pharmaceutical compositions that can be used to treat skin damage or wounds.

In the present invention, the term "individual" means any animal such as rats, mice, and livestock, including humans, that has or may develop skin damage or wounds. The animal may be a human as well as a mammal, such as a cow, horse, sheep, pig, goat, camel, antelope, dog, or cat, in need of prevention or treatment of similar symptoms thereto, but is not limited thereto.

The composition of the present invention can be administered in a pharmaceutically effective amount.

The term "pharmaceutically effective amount" means an amount sufficient to treat a disease with a reasonable benefit/risk ratio applicable to medical treatment, and the effective dosage level may be determined based on factors including the type and severity of the individual, age, sex, activity of the drug, sensitivity to the drug, time of administration, route of administration and rate of excretion, period of treatment, and concurrently used drugs, and other factors well known in the medical field.

The composition may be administered as an individual therapeutic agent or in combination with other therapeutic agents, and may be administered sequentially or simultaneously with conventional therapeutic agents. The composition may be administered a single time or multiple times. It is important to administer the composition in a minimum amount that can afford the maximum effect as well as does not cause side effects, taking all the factors into consideration, and this minimum amount can be easily determined by a person skilled in the art.

The composition may be administered orally or parenterally (for example, intravenously, subcutaneously, intraperitoneally, or topically) depending on the desired method, and the dosage varies depending on the patient's condition and weight, degree of disease, drug form, and route and time of administration, but may be appropriately selected by a person skilled in the art. As a specific example, the composition can generally be administered one time or several times a day, but the preferred dosage may be appropriately selected by a person skilled in the art depending on the individual's condition and weight, degree of disease, drug form, and route and period of administration.

Still another object of the present invention is to provide a quasi-drug composition for skin regeneration or wound improvement, containing the skin-derived stem cells, a culture of the skin-derived stem cells, or cells differentiated from the skin-derived stem cells as an active ingredient.

In the present invention, the term "quasi-drug composition" is an article used for the purpose of treating, alleviating, healing, or preventing diseases in humans or animals, which corresponds to one of textiles, rubber products or similar articles; articles that weakly act on the human body or do not directly act on the human body and are not instruments or machines and similar articles; and preparations used for sterilization, deinsectization, and similar purposes to prevent infection, and refers to articles used for the purpose of diagnosing, treating, alleviating, healing or preventing diseases in humans or animals excluding those that are not instruments, machines or devices and articles used for the purpose of having a pharmacological effect on the structure and function of humans or animals excluding those that are not instruments, machines, or devices, and may specifically be a skin preparation for external use or a personal care product.

Still another object of the present invention is to provide a cosmetic composition for skin regeneration or wound improvement, containing the skin-derived stem cells, a culture of the skin-derived stem cells, or cells differentiated from the skin-derived stem cells as an active ingredient.

In the present invention, the term "cosmetic composition" may be prepared in any commonly prepared formulation, for example may be formulated as a solution, emulsion, suspension, paste, cream, lotion, gel, powder, spray, surfactant-containing cleansing product, oil, soap, liquid detergent, bath salt, foundation, makeup base, essence, toner, foam, pack, softening water, sunscreen cream, or sun oil.

Still another object of the present invention is to provide a composition for skin-derived stem cell culture, containing skin tissue and a hydrogel, and a kit for skin-derived stem cell culture, containing the composition.

In addition, the kit for skin-derived stem cell culture may include the composition for stem cell culture and various components such as solutions and devices necessary for culture of the stem cells.

Still another object of the present invention is to provide a use of a composition containing the skin-derived stem cells, a culture of the skin-derived stem cells, or cells differentiated from the skin-derived stem cells as an active ingredient for skin regeneration or wound improvement.

### [Mode for Carrying Out the Invention]

Hereinafter, the present invention will be described in more detail with reference to Examples. However, these Examples are for illustrative purposes only, and the scope of the present invention is not limited to these Examples.

### Example 1. Three-dimensional culture of fibrin hydrogel-supported skin tissue

Using skin tissue extracted from an animal or donated from a human as a sample, the skin tissue was treated at 60°C for 1 minute to isolate the stratum corneum, immersed in isopropyl alcohol for 5 minutes to remove the fat layer of the skin, and then washed with PBS (Gibco) three times for pretreatment of the skin tissue. The treated tissue was fixed using forceps, cut into pieces of 1 to 3 mm² using a razor blade, and washed with PBS three times. After washing with DMEM (low glucose, Gibco) was performed once, the supernatant was removed, and 5 mL of fibrinogen solution (0.25% w/v fibrinogen (GC Biopharma) and 200 µg/mL tranexamic acid (Shin Poong Pharmaceutical) in DMEM) was added. After an equal amount of thrombin solution (0.5 IU/mL thrombin (GC Biopharma) in DMEM) was added, the mixture was immediately transferred to a 100 mm culture vessel and gently rolled to spread the tissue fragment and hydrogel evenly. When necessary, forceps were used to secure tissue fragment spacing. Sufficient gelation was achieved by performing culture at room temperature for 5 minutes and at 37°C for 2 hours. A growth medium (90% low DMEM : F12 = 1 : 1, 10% fetal bovine serum, 20 ng/mL epidermal growth factor, 5 ng/mL basic fibroblast growth factor, 10 ng/mL insulin-like growth factor, and 10 mg/mL gentamicin) containing 100 µg/mL tranexamic acid was added by 10 mL, and the culture medium was replaced every 1 hour for 3 hours. After that, the growth medium was changed once every 2 to 3 days.

### Example 2. Recovery and amplification culture of HB-skin-derived stem cells migrated into and grown in fibrin hydrogel

Hair follicle tissue was cultured for about 10 days by the method described in Example 1. After the hydrogel was washed with warm PBS three times for 3 minutes, 30% FBS/DMEM was added, and stirring was performed on an orbital shaker (30 rpm). After the supernatant was removed 1 hour later, 10 mL of 30% FBS/low DMEM supplemented with 3,000 units urokinase (GC Biopharma) was added, and the gel was decomposed. When the gel was sufficiently decomposed, culture was performed overnight under static conditions, then the supernatant was removed, the growth medium was added, and culture was performed in a conventional manner. The growth medium was replaced every 2 to 3 days, and subculture was performed every 4 to 6 days.

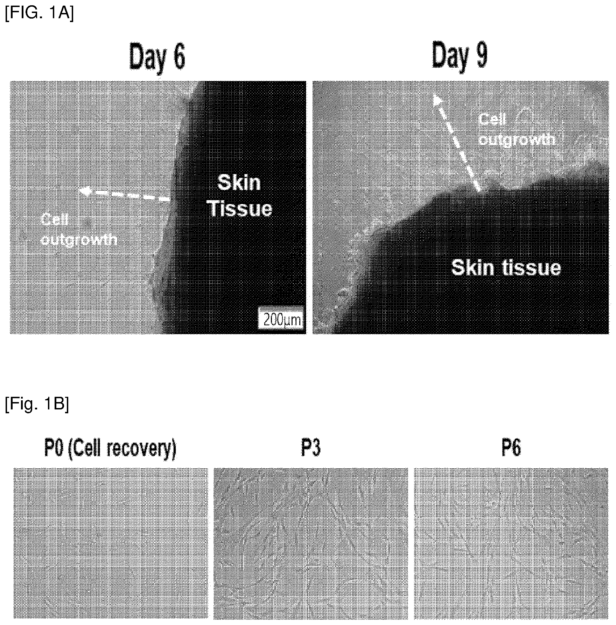

The results obtained by performing tissue culture and cell isolation and culture by the methods are illustrated in FIG. 1.

Specifically, when cells were isolated and subcultured after tissue culture, it was found that the shape and size of the cells were maintained until the ninth subculture.

### Example 3. Examination of immunophenotype of isolated and cultured HB-skin-derived stem cells

In order to examine the immunophenotype of HB-skin-derived stem cells isolated and cultured by the method of Example 2, cells cultured in a T75 flask were washed with PBS once, and then treated with 3 mL of TrypleExpress (Gibco) for 3 minutes to strip the cells. To the stripped cells, 6 mL of 10% calf serum/PBS was added to inhibit the proteolytic reaction. After centrifugation (300 g, 5 min), the supernatant was removed, and the cells were suspended in 3% bovine serum albumin (sigma)/PBS.

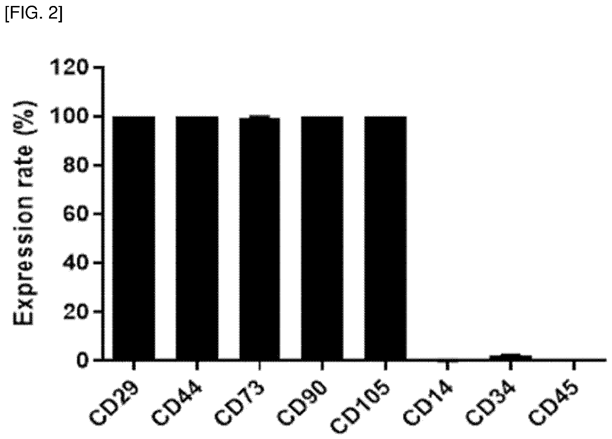

The cell suspension was divided into 9 equal parts and reacted with the following antibodies at 4°C for 1 hour: FITC-conjugated CD29 (Bio-rad, 1 :10), CD44 (Invitrogen, 1:100), CD90 (Invitrogen, 1:20), CD14 (Invitrogen, 1:20) antibody, AlexaFluor488-conjugated CD105 (abcam, 1 :25), PE-conjugated CD73 (Invitrogen, 1:20) antibody, PerCP-conjugated CD34 (Santacruz, 1:5) antibody, or AlexaFluor594-conjugated CD45 (Novus, 1:20) antibody. After the reaction, the reaction mixture was centrifuged at 300 g for 5 minutes, washed, suspended in 3% BSA/PBS, and subjected to measurement using a flow cytometer (BD) (FIG. 2).

As a result, as can be seen from FIG. 2, it was found that CD29, CD44, CD73, CD90, and CD105 were expressed but CD14, CD34, and CD45 were not expressed on the surface of the isolated and cultured HB-skin-derived stem cells.

Specifically, it was found that CD29, CD44, CD90, CD73, and CD105, mesenchymal stem cell markers, were positively expressed (95% or more) but CD34, CD45, and HLA-DR, markers of hematopoietic cells, were negatively expressed (less than 5%).

### Example 4. Examination of proliferation ability and colony formation ability of isolated and cultured HB-skin-derived stem cells

In order to examine the proliferation ability of the isolated and cultured HB-skin-derived stem cells obtained in Example 2, the population doubling time depending on the number of passages was measured through the number of cells obtained by seeding cells at a density of 4000 cells/cm² at the time of initial culture and subculturing the cells every 4 days.

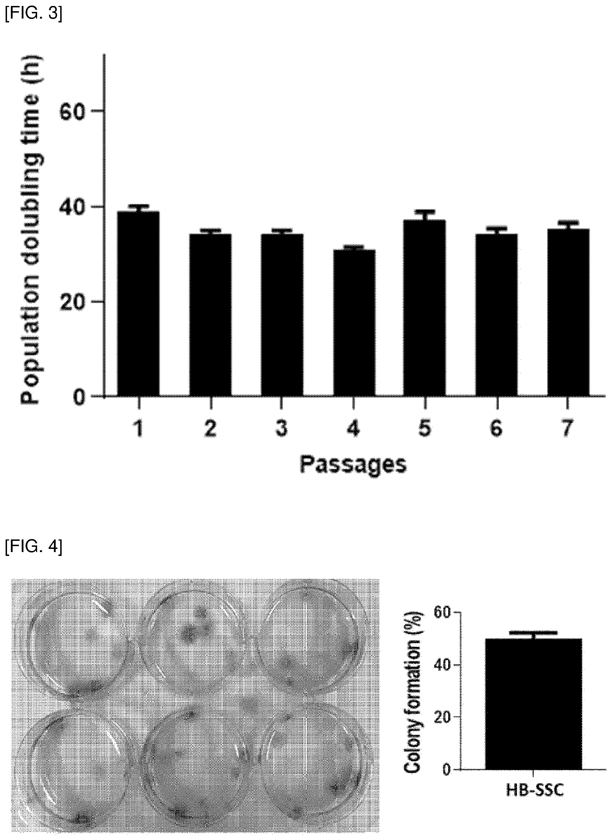

The number of cells was measured by counting the number of unstained cells by trypan blue staining, and the population doubling times (PDTs) were calculated from the following equation and illustrated in FIG. 3: PDT = [days / (logN₂ - logN₁)] / log2, (N₁ = 4,000 cells, N₂ = number of cells after 4 days).

In order to examine the colony formation ability of the isolated and cultured HB-skin-derived stem cells obtained in Example 2, human skin-derived stem cells (4 passages) were seeded in a 6-well plate at 20 cells/well. The cells were cultured for 14 days while the growth medium was replaced every 3 days. The cells were washed with PBS and fixed with 4% paraformaldehyde (4% PFA, Sigma) at room temperature for 15 minutes. The fixed cells were washed with PBS three times and then stained with 0.5% crystal violet/methanol at room temperature for 15 minutes. After the remaining solutions were all removed, washing with distilled water was performed three times, the stained colonies were observed, and the results are illustrated in FIG. 4.

As illustrated in FIGS. 3 and 4, it was found that in the case of HB-skin-derived stem cells obtained by the method of Example 2, when the HB-skin-derived stem cells were subcultured up to the seventh passage and the population doubling time (PDT) was determined, the PDT was observed to be an average of 35 hours ± 6 hours, the proliferation ability was maintained when subculture was performed, and the colony formation ability was also a certain level or more.

### Example 5. Examination of differentiation potency of HB-skin-derived stem cells

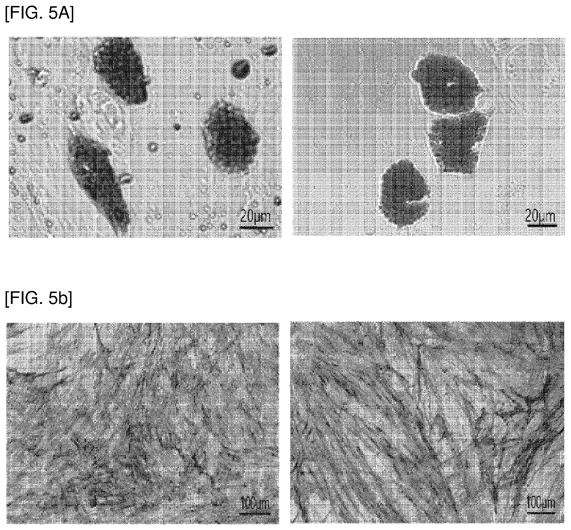

In order to evaluate the differentiation potency of the obtained HB-skin-derived stem cells into adipocytes, cells were seeded at a concentration of 1 × 10⁴ cells/cm² and cultured in a growth medium until 80% confluency was achieved, then the medium was changed to StemPro^{™} Adipogenesis differentiation kit (Gibco, cat. A1007001), and culture was performed for 14 days while the medium was changed every 3 days. The cells were fixed with 4% PFA for 15 minutes and reacted with 60% isopropanol for 15 minutes, then the solutions were all removed, and the cells were thoroughly dried. The cells were stained with 60% Oil Red O (sigma)/ethanol at room temperature for 15 minutes to examine whether there was fat accumulation in the cytoplasm (FIG. 5A).

In order to evaluate the differentiation potency of the HB-skin-derived stem cells into osteocytes, cells were seeded at a concentration of 1 × 10⁴ cells/cm² and cultured in a growth medium until 80% confluency was achieved, then the medium was changed to StemPro^{™} Osteogenesis differentiation kit (Gibco, cat. A1007201), and culture was performed for 14 days while the medium was changed every 3 days. The cells were fixed with 4% PFA for 60 seconds, treated with BCIP/NBT substrate solution (Sigma), and stained for 10 minutes under light-blocking conditions to examine the activity of alkaline phosphatase (ALP) (FIG. 5B).

In other words, from the results, it was verified that HB-skin-derived stem cells obtained by the culture method of the present disclosure possessed pluripotency.

### Example 6. Examination of differentially expressed genes in HB-skin-derived stem cells

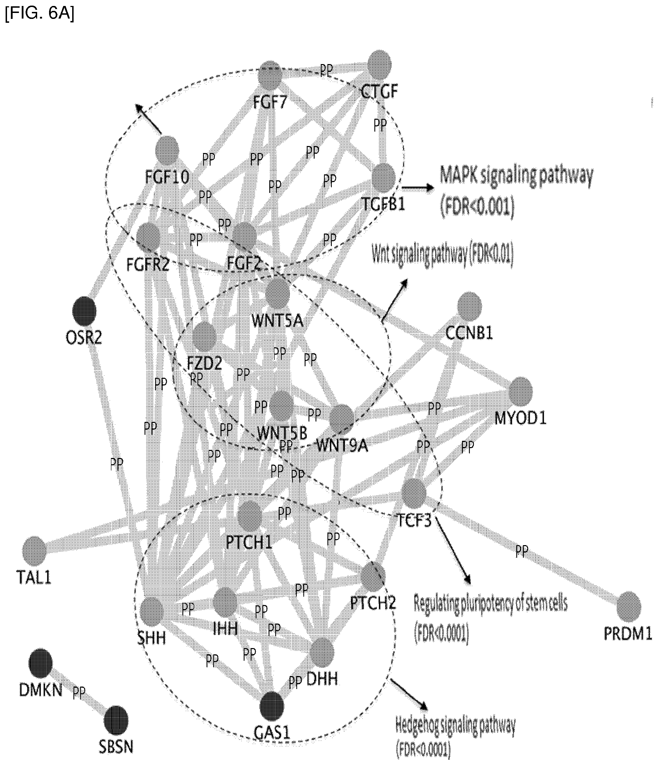

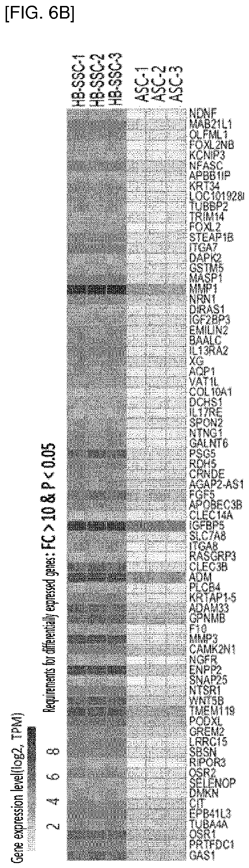

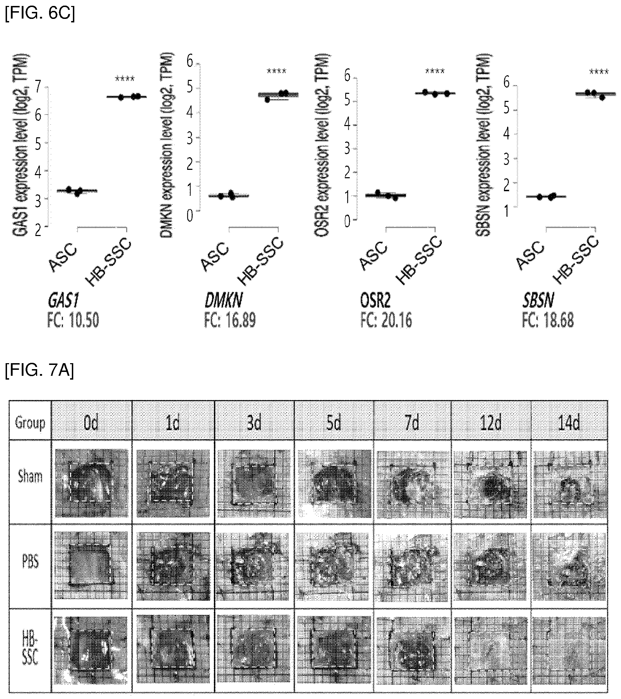

For samples of adipose stem cells (ASC-1. ASC-2. ASC-3) and HB-skin-derived stem cells (HB-SSC-1, HB-SSC-2, HB-SSC-3), a paired-end RNA library was constructed using the TruSeq Stranded mRNA Library Prep Kit according to the manufacturer's protocol, and sequencing was performed at Macrogen (Seoul, Korea). After the pretreatment process, the genes were mapped to the reference genome (version GRCh38) with the HISAT2 program (version 2.1.0, https://ccb.jhu.edu/software/hisat2/index.shtml), and the expression profile for each transcript was acquired using the StringTie program (version 2.1.3b, https://ccb.jhu.edu/software/stringtie/). Gene expression levels were handled by taking the logarithm of TPM (Transcripts Per Kilobase Million) values. Differentially expressed genes between the control and comparison groups were acquired using the edgeR package [1] of the R program (version 4.0) (1. ROBINSON, Mark D.; MCCARTHY, Davis J.; SMYTH, Gordon K. edgeR: a Bioconductor package for differential expression analysis of digital gene expression data. Bioinformatics, 2010, 26.1: 139-140.) (FIGS. 6B and 6C). To construct a biological network in which marker genes participate, a protein-protein interaction (PPI) network was constructed using String Database (version 11.5) [2] (2. SZKLARCZYK, Damian, et al. STRING v11: protein-protein association networks with increased coverage, supporting functional discovery in genome-wide experimental datasets. Nucleic acids research, 2019, 47.D1: D607-D613). The organism reference used to construct the PPI network was Homo sapiens. As settings for constructing a network, the edges connecting nodes representing proteins to each other were connected based on evidence such as experimental results, biological databases and literature, and gene fusion, and an edge score of 0.4 or more was considered significant. After construction of the network, orphan nodes that had no connectivity between other nodes were removed. Only statistically significant biological functions to which node groups belong were extracted based on the KEGG database [3] (3. KANEHISA, Minoru; GOTO, Susumu. KEGG: Kyoto Encyclopedia of Genes and Genomes. Nucleic acids research, 2000, 28.1: 27-30) (False Discovery Rate < 0.05) (FIG. 6A). The test of significant difference between two groups was obtained by performing a T-Test, and a P value < 0.05 was considered significant. The statistical test results provided by the analysis program were used as is, and a significance level of less than 0.05 was considered significant. In a case of indicating results with a P value exceeding 0.05, the P value was indicated separately.

Using String DB, genes expressed in HB-skin-derived stem cells were analyzed with the skin regeneration-related gene network to identify relevant signaling pathways, and it was found that four skin regeneration functional genes (GAS1, OSR2, DMKN, and SBSN) were expressed at high levels (FIG. 6A). In the HB-skin-derived stem cells, 73 genes expressed with fold change > 10 compared to those in adipose-derived stem cells were identified, and among these, four skin regeneration functional genes (GAS1, OSR2, DMKN, and SBSN) derived in FIG. 6A were found to be differentially expressed by 10-fold or more compared to those in adipose-derived stem cells (FIGS. 6B and 6C).

The four skin regeneration functional genes are known to be genes associated with immune regulation and cell proliferation in relation to skin regeneration and wound healing, and based on this, the skin regeneration and wound healing effects of skin-derived stem cells prepared by the skin-derived stem cell preparing method of the present invention can be inferred.

### Example 7. Full-thickness skin wound mouse model and examination of wound healing effect

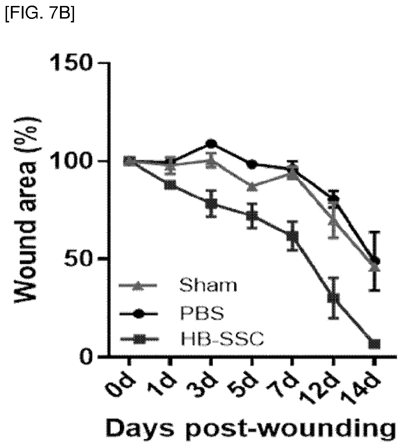

The experimental animals, 12-week-old BALB/c nude mice (Central Laboratory Animal), were anesthetized by inhalation with 3% isoflurane (Hana Pharmaceuticals), and the surgical site was disinfected with an alcohol swab (Meditop). A sticker with a ruler was attached to the dorsal part, then the skin was incised in a square (5 mm × 5 mm, 25 mm²) shape down to the subcutaneous layer with sterilized surgical scissors, and the subcutaneous tissue was isolated to create a full-thickness skin wound. In order to prevent wound contraction, the site around the wound was fixed with Suture (Prolene^{™}, EHICON, cat. W8566). In 100 µL of normal saline (NS), 5 × 10⁵ cells were suspended, and the suspension was subcutaneously (Insulin syringe, 31 gauge) injected to the site around the wound at 25 µL/site (4 sites). After wounding, the surrounding site was disinfected with an alcohol swab to prevent infection, and all experimental groups were observed separately. The wound site of each group was photographed using a digital camera at the same time on days 0, 1, 3, 5, 7, 12, and 14 after wounding, and the wound area was measured using Image J Software (US National Institute of Health Program, USA). The wound area was calculated as a percentage of the wound area for each measurement day based on the wound area immediately after wounding (FIGS. 7A and 7B).

From the results, the wound healing effect of HB-skin-derived stem cells prepared by the preparation method of the present invention was verified.

### Example 8. Examination of skin and skin structure regeneration ability in full-thickness skin wound mouse model

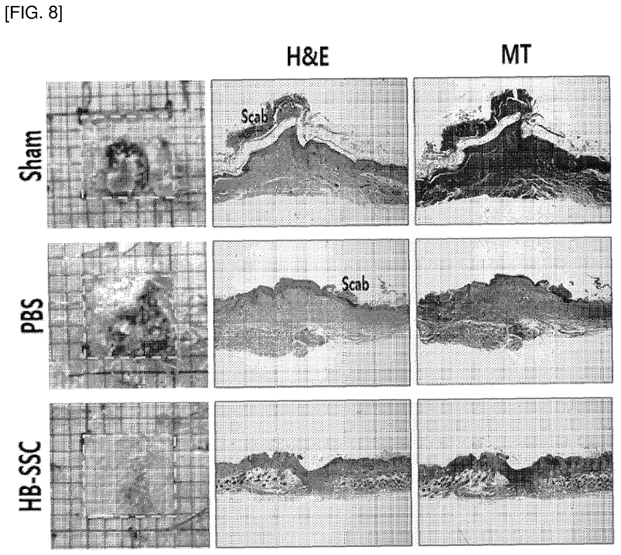

For histological analysis, skin tissue was fixed in a fixative (4% formaldehyde, Sigma, USA) for 24 hours. Thereafter, the tissue was paraffin embedded, the blocks were sectioned to 6 µm, and H&E (Hematoxylin & Eosin) staining was performed. The tissue for each experimental group was observed under an optical microscope, and the results were compared with each other (FIG. 8).

From the results, the skin and skin structure regeneration effect of HB-skin-derived stem cells prepared by the preparation method of the present invention was verified.

Based on the above description, it will be understood by those skilled in the art to which the present invention pertains that the present invention may be implemented in other specific forms without changing the technical spirit or essential features thereof. Therefore, it should be understood that the embodiments described above are not restrictive but illustrative in all aspects. The scope of the present invention is defined by the appended claims rather than by the description preceding them, and therefore all changes and modifications that fall within metes and bounds of the claims or equivalents of such metes and bounds are intended to be embraced by the claims of the present invention.

## Claims

1. A method for culturing skin-derived stem cells, the method comprising:
(a) encapsulating skin tissue in a hydrogel and culturing the skin tissue to obtain a culture; and
(b) decomposing the hydrogel in the obtained culture to recover stem cells that have migrated from the skin tissue into the hydrogel and proliferated in the hydrogel.

2. The method for culturing skin-derived stem cells according to claim 1, wherein the culture of skin tissue in step (a) involves encapsulating a skin fragment in a hydrogel and culturing the skin fragment for 3 to 20 days.

3. The method for culturing skin-derived stem cells according to claim 1, comprising a pretreatment step of skin tissue, which includes treating skin tissue sample at a temperature of 50°C to 70°C for 30 to 90 seconds; immersing the skin tissue sample in an alcohol or a surfactant for 1 to 10 minutes; and performing washing as a pretreatment step of skin tissue before step (a).

4. The method for culturing skin-derived stem cells according to claim 1, wherein the skin-derived stem cells have increased expression of one or more genes selected from GAS1, OSR2, DMKN, or SBSN.

5. The method for culturing skin-derived stem cells according to claim 1, wherein the skin-derived stem cells have immunological characteristics in which CD29, CD44, CD73, CD90, and CD105 are expressed but CD14, CD34, and CD45 are not expressed.

6. The method for culturing skin-derived stem cells according to claim 1, wherein the skin-derived stem cells maintain a cell shape and a cell size until ninth subculture after tissue culture and cell isolation.

7. Skin-derived stem cells prepared by the method according to claim 1.

8. The skin-derived stem cells according to claim 7, wherein the skin-derived stem cells have increased expression of one or more genes selected from GAS1, OSR2, DMKN, or SBSN.

9. The skin-derived stem cells according to claim 7, wherein the skin-derived stem cells have immunological characteristics in which CD29, CD44, CD73, CD90, and CD105 are expressed but CD14, CD34, and CD45 are not expressed.

10. A pharmaceutical composition for skin regeneration or wound treatment, comprising skin-derived stem cells prepared by the method according to claim 1, a culture of the skin-derived stem cells, or cells differentiated from the skin-derived stem cells as an active ingredient.

11. A quasi-drug composition for skin regeneration or wound improvement, comprising skin-derived stem cells prepared by the method according to claim 1, a culture of the skin-derived stem cells, or cells differentiated from the skin-derived stem cells as an active ingredient.

12. A cosmetic composition for skin regeneration or wound improvement, comprising skin-derived stem cells prepared by the method according to claim 1, a culture of the skin-derived stem cells, or cells differentiated from the skin-derived stem cells as an active ingredient.
